# EUROPÄISCHE PATENTANMELDUNG

(11) **EP 1 618 859 A1**
(43) Veröffentlichungstag der Anmeldung: **25.01.2006**
(21) Anmeldenummer: 05015751.0
(22) Anmeldetag: 20.07.2005
(51) Int. Cl.: A61F 9/02, G02C 9/02, G02C 3/00

(54) **Brille, insbesondere Sportbrille**

(30) Priorität: 21.07.2004 DE 202004011798 U
(71) Anmelder: Krauter, Manfred, 01900 Bretnig (DE)
(72) Erfinder: Krauter, Manfred, 01900 Bretnig (DE)
(74) Vertreter: Uhlemann, Henry

(57) **Zusammenfassung**

Die erfindungsgemäße Brille umfasst einen Stirnbügel zur Anlage an der Stirn des Trägers, mit dem Stirnbügel verbundene Befestigungsmittel zur Befestigung des Stirnbügels am Kopf sowie ein optisches Element zum Schutz der Augen oder/und zur Filterung einfallenden Lichts, wobei das optische Element mittels Lagerungsmitteln zur schwenkbaren Lagerung in eine außerhalb des Gesichtsfelds liegende Position über den Stirnbügel hinweg schwenkbar mit dem Stirnbügel verbunden ist.

## Beschreibung

Die Erfindung betrifft eine Brille, insbesondere eine Sportbrille zur Verwendung im Skisport.

Sportbrillen für verschiedenste Einsatzzwecke sind seit langem bekannt und werden in großer Vielfalt hergestellt und angeboten. Bei Spartbrillen, insbesondere Sportbrillen zum Einsatz in Wintersportarten, stellt sich immer wieder das Problem des Scheibenbeschlags auf Grund der Temperaturunterschiede zwischen Innen- und Außenseite des Brillenglases, Zudem ist es für den Athleten, beispielsweise beim Biathlon, nachteilig, das Sichtfenster einer herkömmlichen Skibrille vor den Augen zu haben, da durch die damit unvermeidlich einhergehende optische Verzerrung die Zielgenauigkeit beim Schießen beeinträchtigt wird. Das vorübergehende Absetzen der Brille auf oder oberhalb der Stirn ist keine befriedigende Lösung, da während dieser Zeit das Sichtfenster durch Dampf, der durch die Mütze nach außen dringt, beschlägt, so dass eine anschließende Weiterfahrt verzögert wird. Viele Lösungsvorschläge zur Behebung dieser Probleme sind bekannt geworden.

So wird in DE 6906999 U1 eine Sportbrille, insbesondere Skibrille vorgeschlagen mit einem umlaufenden Rahmen, vor dessen Öffnung ein Sichtfenster angeordnet ist, bei der das Fenster unter Übergreifen der Oberseite des Rahmens ausschwenkbar ist. Diese Sportbrille ist den üblichen einfachen Ausführungsformen von Sportbrillen nachempfunden, ermöglicht aber zusätzlich ein Aufwärtsschwenken des Sichtfensters, um die Innenseite des Sichtfensters beispielsweise in einer Pause ausreichend belüften zu können. Nachteilig an dieser Lösung ist, dass der umlaufende Rahmen auch dabei im Kontakt mit dem Gesicht des Trägers verbleibt, so dass gestaute Nässe zwischen dem Rahmen und dem Gesicht nicht entweichen kann. Weiterhin wird durch den auf dem Gesicht verbleibenden Rahmen das Auf- und Absetzen einer Korrekturbrille erschwert.

Auch die bekannt gewordenen Lösungen, die eine schwenkbare Verbindung zwischen dem Rahmen und der Sichtscheibe einer Brille vorsehen, sind zur Lösung der eingangs geschilderten Probleme nicht geeignet. In DE 93 12 914 U1 ist dazu eine drehbare Verbindung zwischen den Bügeln und dem Scheibenrahmen vorgesehen. Hier wie auch bei den Lösungen gemäß DE 92 07 109 U1 oder EP 0 571 765 A1, bei denen die Sichtscheibe auf Zapfen drehbar gelagert ist, die an einem Rahmenteil mit seitlichen Ansätzen angeordnet sind, ist es unmöglich, die Sichtscheibe aus dem Gesichtsfeld des Trägers zu entfernen, ohne die Brille abzusetzen. Zweck der genannten Lösungen ist es, eine Enstellbarkeit der Bügelneigung relativ zur Sichtscheibe zu ermöglichen, um unterschiedlichen anatomischen Gegebenheiten Rechnung zu tragen.

In DE 20 2004 002 494 U1 wird demgegenüber eine Brille mit einer Sichtscheibe und mit dieser verbundenen Ohrenbügeln und mit einem Nasensteg vorgeschlagen, bei der der Nasensteg am Nasenrücken des Trägers angelenkt ist und mit der Sichtscheibe über eine an dieser über eine gelenkartige Verbindung angelenkte Brücke verbunden ist und die Sichtscheibe in Bezug auf das Gesicht des Trägers nach oben und nach unten bewegbar ist. Die Bewegung wird dadurch erzeugt, dass die gesamte Brille nach oben bzw. anschließend wieder nach unten verschoben wird. Bei dieser Bewegung soll der Nasensteg in Kontakt mit dem Nasenrücken bleiben. Dadurch müssen die Bügel zwangsläufig auf den Ohren hin- und herrutschen, oder der Kontakt zwischen Nasensteg und Nasenrücken ist nicht gewährleistet.

In einer Ausführungsform ist vorgesehen, dass die Neigung der Bügel bezüglich der Sichtscheibe veränderlich ist, wodurch das beschriebene Problem sich noch verschärft, da die Brille mit den Bügeln einen viel größeren Schwenkradius aufweist als die Brücke. Daher und weil die vorgeschlagene Brille Bügel aufweist, die keinen ausreichend sicheren Halt am Kopf ermöglichen, ist die vorgeschlagene Brille als Sportbrille für Skisportarten, insbesondere Biathlon, ungeeignet.

Die Aufgabe der Erfindung besteht daher darin, eine Brille, insbesondere eine Sportbrille anzugeben, die es ermöglicht, die Sichtscheibe oder den vorderen Fassungsteil, der ein oder zwei Scheiben oder Gläser enthalten kann, aus dem Gesichtsfeld des Trägers herauszuschwenken, ohne die Brille abnehmen zu müssen und ohne dass die Gefahr des Beschlagens besteht.
Efindungsgemäß wird die Aufgabe gelöst durch eine Brille, insbesondere eine Sportbrille mit den Merkmalen des Anspruchs 1. Vorteilhafte Ausgestaltungen der Erfindung sind Gegenstand von Unteransprüchen.

Die erfindungsgemäße Brille umfasst einen Stirnbügel zur Anlage an der Stirn des Trägers, mit dem Stirnbügel verbundene Befestigungsmittel zur Befestigung des Stirnbügels am Kopf sowie ein optisches Element zum Schutz der Augen oder/und zur Filterung einfallenden Lichts, wobei das optische Element mittels Lagerungsmitteln zur schwenkbaren Lagerung in eine außerhalb des Gesichtsfelds liegende Position über den Stirnbügel hinweg schwenkbar mit dem Stirnbügel verbunden ist.

Die erfindungsgemäße Brille ermöglicht ein Aufwärtsschwenken des optischen Elements aus dem Gesichtsfeld des Trägers, ohne die Brille abzusetzen. Der Stirnbügel liegt dabei an der Stirn des Trägers an, um das optische Element abzustützen und eine Positionsveränderung der Brille in jeder Schwenkposition überflüssig zu machen. Das Gesicht unterhalb des Stirnbügels liegt nach dem Aufwärtsschwenken des optischen Elements frei, so dass eine Korrekturbrille problemlos aufgesetzt werden kann. Das optische Element dient in der oberen Position weiterhin zur Abschattung des Gesichts, um Blendeffekte zu vermeiden. Gleichzeitig schützt der an der Stirn anliegende Stirnbügel den Augenbereich des Brillenträgers vor dem Eindringen von Schweißtropfen aus dem Stimbereich.

In einer vorteilhaften Ausgestaltung der Erfindung umfasst der Stirnbügel ein Nasenteil zur zusätzlichen Abstützung der Brille auf dem Nasenrücken. Dies hat weiterhin den Vorteil, dass die besonders empfindliche Nase zumindest teilweise vor der Sonne geschützt ist.

Das optische Element kann beispielsweise eine herkömmliche Brillenglasfassung zur Aufnahme zweier Brillengläser sein. In einer vorteilhaften Ausgestaltung der Erfindung umfasst das optische Element einen Schwenkbügel sowie eine an dem Schwenkbügel angeordnete Panoramascheibe, die besonders vorteilhaft aus schlagzähem Kunststoff, beispielsweise Polycarbonat, gefertigt ist. Durch die Verwendung einer solchen Panoramascheibe erweitert sich das Gesichtsfeld des Trägers gegenüber einer herkömmlichen Brillenglasfassung mit zwei Brillengläsern erheblich. Die Panoramascheibe kann als Klarsichtscheibe, in verschiedenen Farben getönt, verspiegelt oder als UV-Filter ausgeführt sein.

In einer vorteilhaften Ausgestaltung der Erfindung ist der Stirnbügel gesichtsseitig mit einer elastischen, schweißabsorbierenden Beschichtung versehen, so dass von der Stirn herablaufende Schweißtropfen vollständig aufgenommen werden und den Träger nicht behindern können.

Das oder die Befestigungsmittel zur Befestigung des Stirnbügels am Kopf können beispielsweise herkömmliche Brillenbügel zur Abstützung der Brille auf den Ohren sein. In einer vorteilhaften Ausgestaltung der Erfindung ist das Befestigungsmittel zur Befestigung des Stimbügels am Kopf ein elastisches Band, das insbesondere bei Verwendung der Brille im Sport für einen sicheren Sitz der Brille sorgt und die Anbringung von Werbung ermöglicht.

Die Lagerungsmittel zur schwenkbaren Lagerung des optischen Elements können beispielsweise an seitlichen Bereichen des Stimbügels angeordnete Zapfen sein, auf denen das optische Element mittels dafür vorgesehener Bohrungen schwenkbar gelagert ist. Umfasst das optische Element einen Schwenkbügel sowie eine an dem Schwenkbügel angeordnete Panoramascheibe, so können die Bohrungen vorteilhaft an seitlichen Bereichen des Schwenkbügels angeordnet sein. In gleicher Weise können die Zapfen selbstverständlich auch an seitlichen Bereichen des optischen Elements, also beispielsweise des Schwenkbügels oder der Panoramascheibe, und die Bohrungen an korrespondierenden Bereichen des Stirnbügels angeordnet sein. Vorteilhaft können die Lagerungsmittel zur schwenkbaren Lagerung des optischen Elements Rastmittel umfassen, die ein ungewolltes Herabschwenken des optischen Elements verhindern. Die konkrete Gestaltung der Lagerungsmittel erlaubt eine Vielzahl von Variationen, ohne vorn Grundgedanken der Erfindung abzuweichen.

In einer vorteilhaften Ausgestaltung der Erfindung sind die Lagerungsmittel zur schwenkbaren Lagerung des optischen Elements als Reiblager ausgeführt, die eine stufenlose Schwenkbarkeit des optischen Elements und eine Arretierung in jeder beliebigen Position erlaubt.

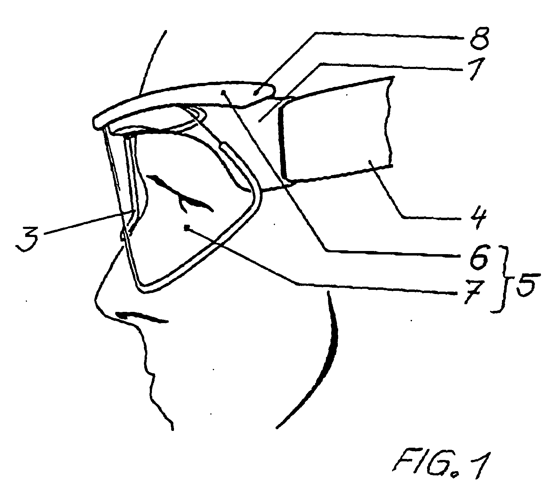

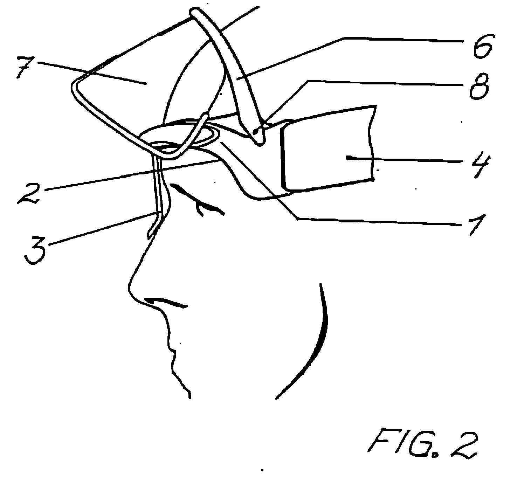

Nachfolgend wird die Erfindung anhand einer Zeichnung näher erläutert Dabei zeigt die Figur ein bevorzugtes Ausführungsbeispiel der Erfindung.

In diesem Ausfuhnmgsbeisplel umfasst die erfindungsgemäße Brille einen Stirnbügel 1 zur Anlage an der Stirn des Trägers, der gesichtsseitig mit einer elastischen, schweißabsorbierenden Beschichtung 2 versehen ist und ein Nasenteil 3 zur zusätzlichen Abstützung der Brille auf dem Nasenrücken aufweist, ein mit dem Stirnbügel 1 verbundenes Befestigungsmittel 4 zur Befestigung des Stimbügels 1 am Kopf und ein optisches Element 5 zum Schutz der Augen und zur Filterung einfallenden Lichts, das einen Schwenkbügel 6 und eine Panoramascheibe 7 umfasst und das mittels zweier Lagerungsmittel 8 in eine außerhalb des Gesichtsfelds liegende Position über den Stirnbügel 1 hinweg schwenkbar mit dem Stirnbügel 1 verbunden ist.

Das optische Element 5 umfasst einen Schwenkbügel 6 und eine Panoramascheibe 7, die aus dem schlagzähen Kunststoff Polycarbonat gefertigt und als UV-Filter ausgeführt ist. Das Gesichtsfeld des Trägers ist dadurch gegenüber einer herkömmlichen Brillenglasfassung mit zwei Brillengläsern erheblich vergrößert.

Der Schwenkbügel 6 des optischen Elements 5 ist mittels Lagerungsmitteln 8 schwenkbar mit dem Stirnbügel 1 verbunden. Die Lagerungsmittel 8 sind so ausgeführt und angeordnet, dass das optische Element 5, d. h, der Schwenkbügel 6 mit der Panoramascheibe 7, In eine außerhalb des Gesichtsfelds liegende Position über den Stirnbügel 1 hinweg schwenkbar ist.

Der Sümbügel 1 umfasst ein Nasenteil 3 zur zusätzlichen Abstützung der Brille auf dem Nasenrücken. Dadurch wird der Stirnbügel 1 in seiner Funktion unterstützt und die besonders empfindliche Nase ist in diesem Bereich vollständig vor der Sonne geschützt. Gleichzeitig definiert das Nasenteil 3 die untere Position des optischen Elements 5, wenn die Panoramascheibe 7 bei der Abwärtsbewegung in Kontakt mit dem Nasenteil 3 kommt. Dadurch wird auch verhindert, dass die Kante der Panoramascheibe 7 mit dem Gesicht in Berührung kommt, wodurch Scheibenbeschlag sowie die Ausbildung schmerzhafter Druckstellen im Gesicht vermieden werden.

Der Stirnbügel 1 ist gesichtsseitig mit einer elastischen, schweißabsorbierenden Beschichtung 2 versehen, so dass von der Stirn herablaufende Schweißtropfen vollständig aufgenommen werden und dadurch das Sehvermögen des Brillenträgers nicht behindert wird.

Das Befestigungsmittel 4 zur Befestigung des Stimbügels 1 am Kopf ist ein elastisches Band, das bei Verwendung der Brille im Sport für einen sicheren Sitz der Brille sorgt und als Werbeträger dienen kann.

Die Lagerungsmittel 8 zur schwenkbaren Lagerung des optischen Elements 5 sind als Reiblager ausgeführt, die eine stufenlose Schwenkbarkeit des optischen Elements 5 in jede beliebige Position erlauben, ohne dass das optische Element 5 von selbst herabschwenkt. Dabei ist jedes Reiblager 8 zwischen einem seitlichen Bereich des Schwenkbügels 6 des optischen Elements 5 und einem korrespondierenden seitlichen Bereich des Stirnbügels 1 angeordnet und stellt eine schwenkbare Verbindung zwischen dem Schwenkbügel 6 des optischen Elements 5 und dem Stirnbügel 1 her, so dass das optische Element 5, d.h. der Schwenkbügel 6 mit der Panoramascheibe 7, in eine außerhalb des Gesichtsfelds liegende Position über den Stirnbügel 1 hinweg schwenkbar ist.

Die erfindungsgemäße Brille ermöglicht ein Aufwärtsschwenken des optischen Elements 5 aus dem Gesichtsfeld des Trägers, ohne die Brille abzusetzen. Der Stirnbügel 1 liegt dabei stets an der Stirn des Trägers an, um das optische Element 5 abzustützen. Dadurch erübrigt sich eine Änderung der Position der Brille, d. h. des Stimbügels 1 und des Befestigungsmittels 4, relativ zum Gesicht. Das Gesicht unterhalb des Stimbügels 1 liegt nach dem Aufwärtsschwenken des optischen Elements 5 frei, so dass eine Korrekturbrille problemlos aufgesetzt werden kann. Das optische Element 5 dient in der oberen Position weiterhin zur Abschattung des Gesichts, um Blendeffekte zu vermeiden. Gleichzeitig verhindert der ständig an der Stirn anliegende Stirnbügel 1 durch die schweißabsorbierende Beschichtung 2 das Herablaufen von Schweißtropfen.

### Bezugszeichenliste

- 1: Stirnbügel
- 2: schweißabsorbierende Beschichtung
- 3: Nasenteil
- 4: Befestigungsmittel
- 5: optisches Element
- 6: Schwenkbügel
- 7: Panoramascheibe
- 8: Lagerungsmittel

## Patentansprüche

1. Brille, insbesondere Sportbrille, umfassend einen Stirnbügel (1) zur Anlage an der Stirn des Trägers, mit dem Stirnbügel (1) verbundene Befestigungsmittel (4) zur Befestigung des Stimbügels (1) am Kopf sowie ein optisches Element (5) zum Schutz der Augen und/oder zur Filterung einfallenden Lichts, wobei das optische Element (5) mittels Lagerungsmitteln (8) zur schwenkbaren Lagerung in eine außerhalb des Gesichtsfelds liegende Position über den Stirnbügel (1) hinweg schwenkbar mit dem Stirnbügel (1) verbunden ist.

2. Brille nach Anspruch 1, **dadurch gekennzeichnet, dass** der Stirnbügel (1) ein Nasenteil (3) zur zusätzlichen Abstützung der Brille auf dem Nasenrücken umfasst.

3. Brille nach Anspruch 1 oder 2, **dadurch gekennzeichnet, dass** das optische Element (5) einen Schwenkbügel (6) sowie eine an dem Schwenkbügel (6) angeordnete Panoramascheibe (7) umfasst.

4. Brille nach Anspruch 3, **dadurch gekennzeichnet, dass** die Panoramascheibe (7) aus schlagzähem Kunststoff besteht.

5. Brille nach einem der vorhergehenden Ansprüche, **dadurch** gekennz e i c h n e t, d a s s der Stirnbügel (1) gesichtsseitig eine elastische, schweißabsorbierende Beschichtung (2) aufweist.

6. Brille nach einem der vorhergehenden Ansprüche, **dadurch gekennzeichnet, dass** das Befestigungsmittel (4) zur Befestigung des Stimbügels (1) am Kopf ein elastisches Band ist.

7. Brille nach einem der vorhergehenden Ansprüche, **dadurch gekennzeichnet, dass** die Lagerungsmittel (8) zur schwenkbaren Lagerung des optischen Elements (5) an seitlichen Bereichen des Stimbügels (1) und des optischen Elements (5) angeordnete Paarungen von Zapfen und Bohrungen sind.

8. Brille nach einem der vorhergehenden Ansprüche, **dadurch gekennzeichnet, dass** die Lagerungsmittel (8) zur schwenkbaren Lagerung des optischen Elements (5) Rastmittel umfassen.

9. Brille nach einem der vorhergehenden Ansprüche, **dadurch gekennzeichnet, dass** die Lagerungsmittel (8) zur schwenkbaren Lagerung des optischen Elements (5) als Reiblager ausgeführt sind.
